(19) Europäisches Patentamt
European Patent Office
Office européen des brevets

(11) Veröffentlichungsnummer: **0 111 090**
**B1**

(12) # EUROPÄISCHE PATENTSCHRIFT

(45) Veröffentlichungstag der Patentschrift: **18.03.87**

(51) Int. Cl.⁴: **C 07 D 209/16**, C 07 F 9/65, C 07 D 471/04

(21) Anmeldenummer: **83110071.4**

(22) Anmeldetag: **08.10.83**

(54) **Indolderivate, Verfahren zu ihrer Herstellung und deren Verwendung zur Weiterverarbeitung.**

(30) Priorität: **29.10.82 DE 3240513**

(43) Veröffentlichungstag der Anmeldung: **20.06.84 Patentblatt 84/25**

(45) Bekanntmachung des Hinweises auf die Patenterteilung: **18.03.87 Patentblatt 87/12**

(84) Benannte Vertragsstaaten: **AT BE CH DE FR GB IT LI LU NL SE**

(56) Entgegenhaltungen: **EP-A-0 030 254**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Biere, Helmut, Dr., Zeltinger Strasse 15, D-1000 Berlin 28 (DE)**

EP 0 111 090 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue Indolderivate, Verfahren zu ihrer Herstellung und deren Verwendung zur Weiterverarbeitung gemäß der Patentansprüche.

Indolderivate der allgemeinen Formel I gemäß Anspruch 1 sind wertvolle Zwischenprodukte zur Herstellung von ß-Carbolinderivaten der allgemeinen Formel IV und von Tryptophanderivaten der allgemeinen Formel V gemäß Anspruch 7.

Carbolinderivate, insbesondere substituierte ß-Carbolinderivate, haben in letzter Zeit hohes Interesse in der Arzneimittelforschung gefunden, da sie eine Fülle von therapeutisch nutzbaren Wirkungen über das Zentralnervensystem ausüben. Sie entfalten beispielsweise anticonvulsive, anxiolytische und muskelrelaxierende sowie sedierende Effekte.

Die Bedeutung, die dieser Stoffklasse beigemessen wird, spiegelt sich in der großen Zahl der Patentanmeldungen wieder, von denen beispielsweise genannt seien: DE-OS 30 15 816, DE-OS 30 23 567, DE-OS 30 48 318 und US-PS 3 202 667.

Substituierte Tryptophane sind zum Beispiel Verbindungen mit schlafanstoßender Wirkung. Tryptophan findet breite Verwendung , zum Beispiel als Zusatz zu Infusionslösungen, zu Futtermitteln usw.

Der Substituent R in den Formeln I, II, IV und V kann sich in den Stellungen 4, 5, 6 oder 7 des aromatischen Ringes befinden, wobei der Ring mit dem Substituenten R mono- oder disubstituiert sein kann. R kann Wasserstoff, Halogen oder einen beliebigen organischen Rest bedeuten.

Als Halogenatome kommen Fluor, Chlor, Brom und Jod in Frage. Als organische Reste kommen vorzugsweise in Frage: Alkyl-, Alkoxyalkyl- und Alkoxygruppen mit 1 bis 4 Kohlenstoffatomen, Methylendioxy-, Benzyloxy-, Cyano-, Alkoxycarbonyl- und Dialkylaminogruppen mit jeweils 1 bis 4 Kohlenstoffatomen im Alkylteil.

Die in der Literatur beschriebenen Verfahren zur Herstellung von ß-Carbolinen und Tryptophanderivaten haben den Nachteil, daß sie über mehrere Stufen verlaufen und in den Ausbeuten nicht immer befriedigend sind (R.A. Abramovitch and J.D. Spenser, Advances in Heterocycl. Chemistry, Vol. 3, p. 79).

Eine typische Carbolin- und Tryptophansynthese sei anhand des folgenden Formelschemas erläutert:

**Formelschema**

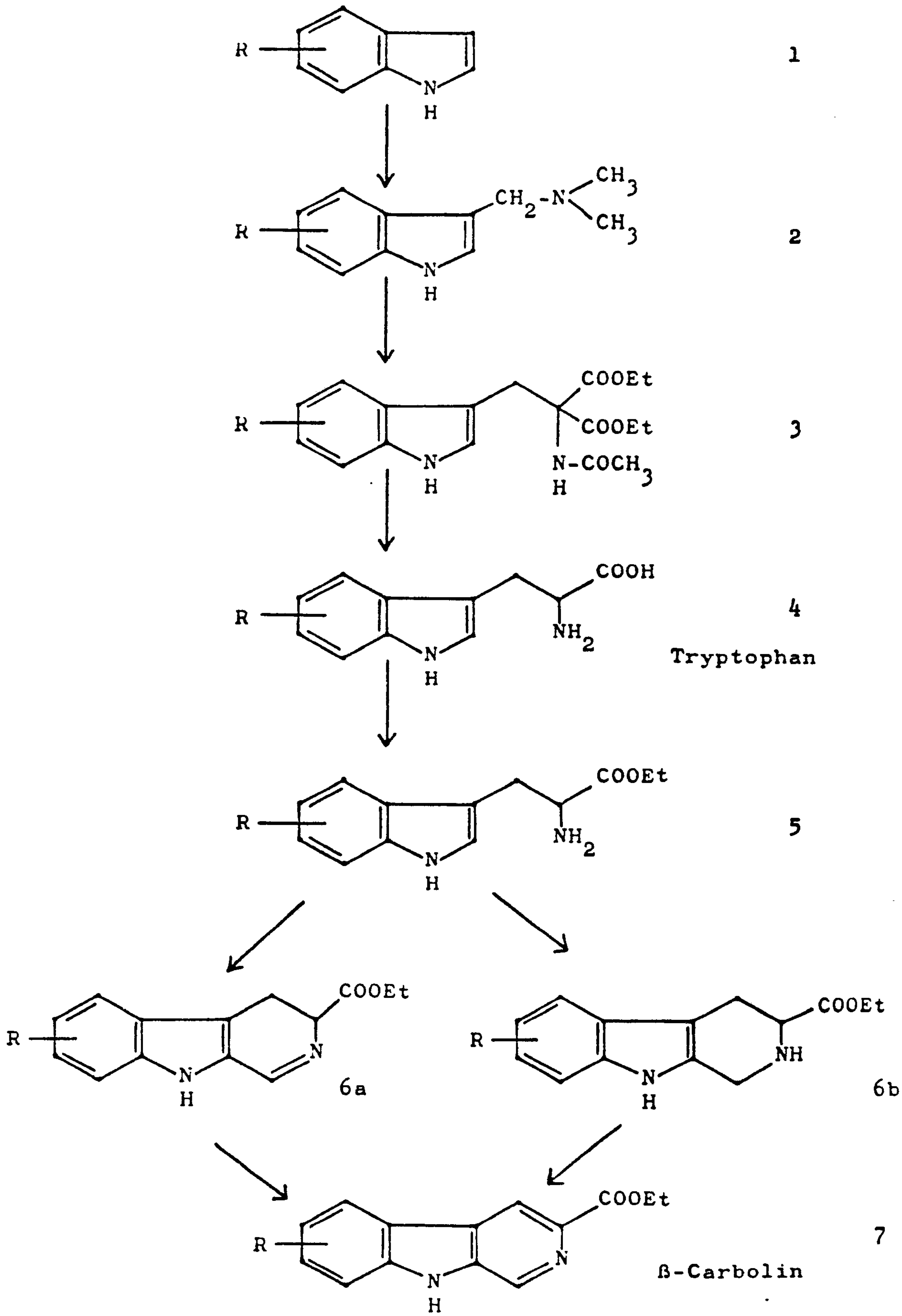

1

2

3

4 Tryptophan

5

6a

6b

7 ß-Carbolin

Ausgehend vom Indol (1) wird mit Formaldehyd und sekundärem Amin Gramin bzw. eine Gramin analoge Verbindung (2) hergestellt, die durch Reaktion mit Acetamidomalonester unter Basenkatalyse in eine Tryptophan-Vorstufe (3) überführt wird.

Nach Abspaltung aller Schutzgruppen und Decarboxylierung entsteht racemisches Tryptophan (4) und nach Veresterung Tryptophanester (5), aus dem nach Acylierung der Aminogruppe und Cyclisierung unter den Bedingungen der Bischler-Napieralski-Reaktion ein 3.4-Dihydro-β-carbolin (6a) und nach Pictet-Spengler ein Tetrahydro-β-carbolin (6b) entsteht, die durch Dehydrierung in die Carboline (7) überführt werden.

Abgesehen von der Vielzahl der Synthesestufen mit den zwangsläufigen Zeit- und Ausbeuteverlusten sind besonders die Cyclisierungen nach Bischler-Napieralski sowie Pictet-Spengler trotz zahlreicher Verfahrensverbesserungen nur in geringer Ausbeute durchführbar, wobei empfindliche, teilhydrierte Zwischenprodukte entstehen, die Anlaß zu verschiedenen Nebenreaktionen geben können. Auch die Dehydrierungsreaktionen an den Di- und Tetrahydrocarbolinen laufen oft in geringer Ausbeute ab.

Aus diesem Grunde wäre es als ein verfahrenstechnisch besonders wichtiger Fortschritt anzusehen, wenn es gelänge, an ungesättigten Indolvorstufen, wie sie zum Beispiel Dehydrotryptophanderivate darstellen, eine Ringschlußreaktion durchzuführen, wobei eine einfache Herstellungsmethode für die benötigten Dehydrotryptophanderivate eine Voraussetzung wäre.

Ein weiterer wichtiger verfahrenstechnischer Fortschritt wäre dann gegeben, wenn es gelänge, diese Ringschlußreaktion an einem Dehydrotryptophanderivat in der Weise durchzuführen, daß anstelle eines 1.2-Dihydrocarbolinderivats das aromatische Carbolinsystem entsteht.

Es ist die Aufgabe der Erfindung, ungesättigte Carbolinvorstufen zu entwickeln, die eine glatte Ringschlußreaktion zu den aromatischen Carbolinen gestatten.

Es wurde nun gefunden, daß die neuen Indolderivate der allgemeinen Formel I einerseits als Dehydrotryptophan Derivate wichtige Zwischenprodukte zur Herstellung von Tryptophan und Tryptophan analogen Verbindungen ($R^4$ = H, $R^3$ = COOAlkyl)sind, da sie durch katalytische Hydrierung, gewünschtenfalls auch durch asymmetrische Hydrierung an literaturbekannten chiralen Rhodium-Katalysatoren, in die optisch aktiven Tryptophan-Vorstufen überführt werden können, die nach Hydrolyse von Ester- und Amidingruppierung Tryptophan bzw. seine Analoga ergeben.

Es wurde ferner gefunden, daß die neuen Indolderivate der allgemeinen Formel 1 andererseits geeignete Vorstufen für eine Cyclisierung zu den gewünschten ß-Carbolinen der allgemeinen Formel IV darstellen, wobei

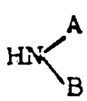

abgespalten wird. Die Cyclisierung erfolgt in guter Ausbeute sowohl bei der thermischen Belastung als auch beim Erhitzen unter katalytischen Bedingungen.

Bei der thermischen Belastung wird das Indolderivat der allgemeinen Formel 1 auf 150 - 250°C, vorzugsweise 180 - 220°C, erhitzt. Eine andere Möglichkeit besteht darin, daß das Indolderivat in einem hochsiedenen Lösungsmittel wie Chinolin oder Diphenylether unter Rückfluß erhitzt wird. Carbolinbildung wird auch beobachtet, wenn das Indolderivat auf Trägermaterial, wie zum Beispiel Kieselgel, mit Hilfe eines leicht verdampfbaren Lösungsmittel, zum Beispiel Dichlormethan, aufgezogen und das substanzbeladene Trägermaterial auf etwa 180-210°C erhitzt wird, was zweckmäßigerweise auch unter Rollen im Kugelrohrofen geschehen kann.

Unter der katalysierten Reaktion zum ß-Carbolin sollen in erster Linie protonenkatalysierte Umsetzungen verstanden werden. Die Umsetzungen werden so ausgeführt, daß das Indolderivat zum Beispiel in organischen Säuren, wie zum Beispiel Ameisensäure, Essigsäure, Propionsäure oder Trifluoressigsäure, oder im anorganischen Milieu, wie zum Beispiel Phosphorsäure, Polyphosphorsäure oder Phosphoroxychlorid usw., erhitzt wird. Es können auch inerte organische Lösungsmittel, wie zum Beispiel Toluol, Ethylacetat, Dioxan, Dimethoxyethan, Acetonitril u.a. als Verdünnungsmittel verwendet werden.

Die Herstellung der erfindungsgemäßen Indolderivate der allgemeinen Formel I erfolgt in einem Schritt durch Umsetzung von Indolen der allgemeinen Formel II mit einem Aza-butadien der allgemeinen Formel III in Gegenwart von Säuren nach Anspruch 5.

Als Lösungsmittel und gleichzeitig Katalysatoren eignen sich besonders flüssige organische Säuren, wie z.B. Ameisensäure, Essigsäure, Propionsäure, Trifluoressigsäure u.a. allein oder im Gemisch mit anderen inerten Lösungsmitteln, wie z.B. Dichlormethan, Tetrahydrofuran, Dioxan, Dimethoxyethan, Essigsäureethylester, Acetonitril, aber auch katalytische Mengen von Mineralsäuren wie Schwefelsäure, Salzsäure, Perchlorsäure etc. in inerten Lösungsmitteln (wie oben) sind verwendbar. Ebenfalls geeignete Katalysatoren und Lösungsmittel stellen Phosphorsäure, Polyphosphorsäure oder Phosphoroxychlorid dar. Je nach gewählten Katalysatoren bzw. Lösungsmitteln liegt die optimale Reaktionstemperatur etwas unterschiedlich, im allgemeinen jedoch liegt diese zwischen 0 und 100°C, in Trifluoressigsäure, z.B. zwischen 0 und 50°C, vorzugsweise bei Raumtemperatur. Die Wahl der Bedingungen richtet sich aber auch nach den im eingesetzten Indol noch vorhandenen Substituenten. Donator-substituierte Indole reagieren im allgemeinen schneller und daher unter milderen Bedingungen als Akzeptor-substituierte Indole.

Die Herstellung der Azabutadiene der allgemeinen Formel III soll anhand der folgenden Beispiele erläutert

werden:

1.) 3-Dimethylamino-2-(dimethylaminomethylenamino)-acrylsäure-ethylester (Azadien 1)

a) gemäß W. Kantlehner et al., Liebigs Ann.Chem. 1980, 344.

b) 3,1 g frisch destillierter Glycin-ethylester wird mit 17,7 g Dimethylformamiddiethylacetal und 0,3 g Kalium-tert.butylat versetzt und zunächst bei 80° C (Badtemperatur), dann allmählich auf 160° C (Badtemperatur) erhitzt, wobei der entstehende Alkohol und andere leichtflüchtige Bestandteile abdestillieren. Nach 5 Stunden wird der Rückstand im Hochvakuum fraktioniert destilliert, dann noch einmal am Kugelrohr destilliert. Ausbeute: 3,6 g (54 %); Kp. 150-160° C (bei 0,05 Torr.), gaschromatographische Reinheit 97 %; $n_D^{25}$ 1,5550.

2.) 3-Dimethylamino-2-(dimethylaminomethylenamino-crotonsäure-ethylester (Azadien 2)

Eine Mischung von 4,7g N (Dimethylaminomethylen)glycin-ethylester (W. Kantlehner et al., Liebigs Ann.Chem. 1980, 344), 8 g Dimethylacetamiddimethylacetal und 0,4 g Kalium-tert.butylat werden analog 1b) 8 Stunden erhitzt unter Abdestillieren des entstehenden Alkohols und anschließend fraktioniert destilliert.

3.) 2-Dimethylamino-1-(dimethylaminomethylenamino)-ethen-phosphonsäure-diethylester (Azadien 3)

Eine Mischung von 3,7 g Aminomethanphosphonsäurediethylester und 15 g des Aminalesters tert. Butoxy-N.N.N'.N''-tetramethylmethandiamin werden 6 Stunden auf ca. 160°C erhitzt. Nach fraktionierter Destillation des Rückstandes im Kugelrohr bei 160-165° C und 0,03 mm erhält man 4,2 g (69 %) der Titelverbindung.

4.) $N^2$-(2-Dimethylamino-1-phenylvinyl)-$N^1$.$N^1$-dimethylformamidin (Azadien 4)

nach W.Kantlehner et al., Liebigs ann.Chem. 1980, 344.

Die folgenden Beispiele sollen das erfindungsgemäße Verfahren näher erläutern.

**Beispiel 1**

2-(Dimethylaminomethylenamino)-3-(3-indolyl)-acrylsäure-ethylester

a) 2,3 g Indol und 6,4 g Azadien 1 werden unter Eiskühlung mit 40 ml Trifluoressigsäure gelöst und 48 Stunden bei Raumtemperatur gerührt. Anschließend wird auf Eiswasser gegossen, mit Natriumhydrogencarbonatlösung neutralisiert und mit Ethylacetat extrahiert.

Nach Einengen der organischen Phase wird der Rückstand über eine Kieselgelsäule mit Ethylacetat chromatographiert und schließlich aus Toluol umkristallisiert. Man erhält 2 g (35 %) produkt vom Schmelzpunkt 138° C.

b) 1,3 g Azadien 1 in 10 ml Dichlormethan gelöst, werden unter Eiskühlung mit 4,5 ml Trifluoressigsäure versetzt. Nach Zugabe von 0,47 g Indol wird 2 Stunden bei 0° C, dann 72 Stunden bei Raumtemperatur gerührt. Nach Aufarbeitung wie oben werden 650 mg (57 %) Produkt vom Schmelzpunkt 137° C (Toluol) erhalten.

**Beispiel 2**

2-(Dimethylaminomethylenamino)-3-(5-benzyloxy-3-indolyl) acrylsäure-ethylester

2,2 g 5-Benzyloxyindol und 3,6 g Azadien 1 werden in 17 ml Eisessig 24 Stunden auf 50° C erwärmt. Nach Aufarbeitung erhält man 2,5 g (65 %) produkt vom Schmelzpunkt 140° C (Toluol).

**Beispiel 3**

2-(Dimethylaminomethylenamino)-3-(4-cyano-3-indolyl-) acrylsäure-ethylester

320 mg Azadien 1 werden mit 2,5 ml Trifluoressigsäure versetzt und 10 Minuten bei Raumtemperatur gerührt. Dann werden 140 mg 4-Cyano-indol zugefügt und die Lösung 1 Stunde unter Stickstoff auf 80° C erhitzt. Nach Aufarbeitung erhält man 140 mg (45 %) produkt vom Schmelzpunkt 159° C (Ethanol).

**Beispiel 4**

1-(Dimethylaminomethylenamino)-2-(3-indolyl)-ethen-phosphonsäure-diethylester

Herstellung erfolgt analog Beispiel 1b aus Indol und Azadien 3.

Die Verwendung von Verbindungen der allgemeinen Formel I zur Herstellung von β-Carbolinen der allgemeinen Formel IV soll anhand der folgenden Arbeitsvorschriften erläutert werden.

1.) β-Carbolin-3-carbonsäure-ethylester

a) 285 mg Indolderivat gemäß Beispiel 1 werden in einer Kugelrohrapparatur unter Anlegen eines Vakuums von ca. 100 Torr 1 Stunde auf 200-210° C erhitzt. Nach Erkalten wird das Reaktionsprodukt in Toluol/Tetrahydrofuran gelöst und über Kieselgel filtriert, schließlich aus Acetonitril umkristallisiert. Man erhält 120 mg (50 %) vom Schmelzpunkt 233-235° C.

b) Der obengenannte β-Carbolinester wird ebenfalls erhalten, wenn das Indolderivat (Beispiel 1), in Chinolin gelöst, 6 Stunden auf 200° C erhitzt wird.

c) Der obengenannte β-Carbolinester wird ebenfalls erhalten, wenn das Indolderivat (Beispiel 1), in wenig Dichlormethan gelöst, auf Kieselgel aufgezogen und dieses Material im Kugelrohr 1 Stunde auf 200° C erhitzt wird.

d) Der obengenannte β-Carbolinester wird ebenfalls erhalten, wenn das Indolderivat (Beispiel 1) in Trifluoressigsäure auf ca 80° C erhitzt wird.

e) Der obengenannte ß-Carbolinester wird ebenfalls erhalten, wenn das Indolderivat (Beispiel 1) in Eisessig unter Rückfluß erhitzt wird.

**Patentansprüche**

1.) Indolderivate der allgemeinen Formel I

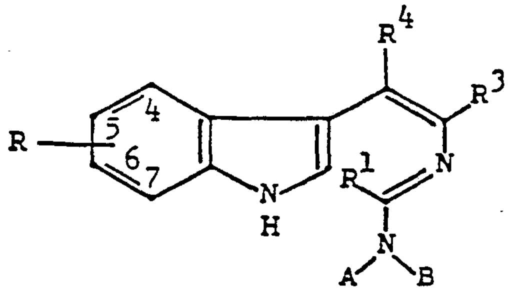

(I),

worin

R Wasserstoff, ein oder zwei Halogenatome oder Alkylengruppe mit 1 bis 4 Kahlenstoffatomen, Alkoxyalkylgruppen mit 1 bis 4 Kohlenstoffatomen, Alkoxygruppen mit 1 bis 4 Kohlenstoffatomen, Methylendioxygruppen, Benzyloxygruppen, Cyanogruppen, Alkoxycarbonylgruppen mit 1 bis 4 Kohlenstoffatomen im Alkylteil oder Dialkylaminogruppen mit 1 bis 4 Kohlenstoffatomen im Alkylteil in der 4-, 5-, 6- oder 7-Stellung,

$R^1$ und $R^4$ gleich oder verschieden sind und Wasserstoff, Alkyl mit 1 bis 3 Kohlenstoffatomen oder Alkoxyalkyl mit jeweils 1 bis 3 Kohlenstoffatomen im Alkoxy- und Alkylteil, $R^3$ Phenyl, -COOAlkyl, $PO_3(Alkyl)_2$, $-SO_2$Aryl, $-SO_2$Alkyl,

$R^3$ Phenyl, -COOAlkyl, $-PO_3(Alkyl)_2$, $-SO_2$Aryl, $-SO_2$Alkyl,

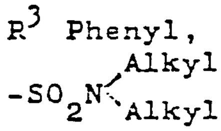 , -CN, 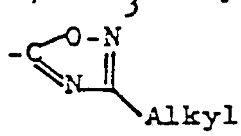 und 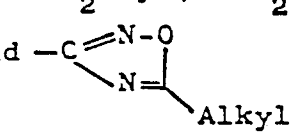

mit jeweils 1 bis 3 Kohlenstoffatomen im Alkylrest und

A und B jeweils für sich Alkyl mit 1 bis 3 Kohlenstoffatomen oder gemeinsam unter Einschluß von N eine Pyrrolidino-, Piperidino-, Morpholino- oder Piperazinogrüppe bedeuten.

2.) 2-(Dimethylaminomethylenamino)-3-(3-indolyl)-acrylsäure-ethylester

3.) 2-(Dimethylaminomethylenamino)-3-(5-benzyloxy-3-indolyl)-acrylsäure-ethylester

4.) 2-(Dimethylaminomethylenamino)-3-(4-cyano-3-indolyl)-acrylsäure-ethylester

5.) 1-(Dimethylaminomethylenamino)-2-(3-indolyl)-ethen-phosphonsäure-diethylester

6.) Verfahren zur Herstellung von Indolderivaten der allgemeinen Formel I, dadurch gekennzeichnet, daß man ein Indol der allgemeinen Formel II

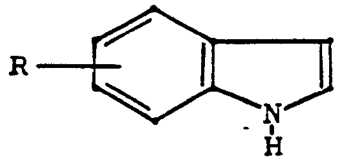

(II),

worin R die in Formel I angegebene Bedeutung hat, mit einem Aza-butadien der allgemeinen Formel III

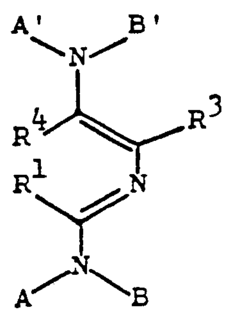

(III),

worin

A' und B' die gleiche Bedeutung wie A und B haben und $R^1$, $R^3$, $R^4$ und A und B die in Formel I angegebene Bedeutung haben, in Gegenwart von Säuren bei Temperaturen von 0 bis 50°C umsetzt.

7.) Verwendung von Verbindungen der allgemeinen Formel I gemäß Anspruch 1 zur Herstellung von

a) ß-Carbolinderivaten der allgemeinen Formel IV

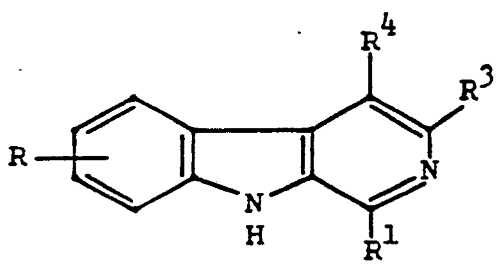

(IV),

worin R, $R^1$, $R^3$ und $R^4$ die in Formel I angegebene Bedeutung haben, und

b) Tryptophanderivaten der allgemeinen Formel V

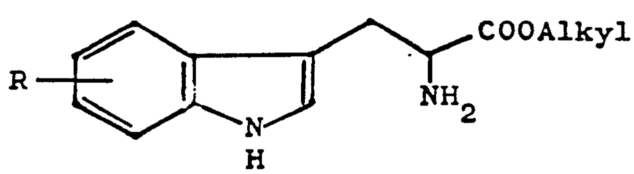

(V),

worin R die in Formel 1 angegebene Bedeutung hat.

**Claims**

1. Indole derivatives of general formula I

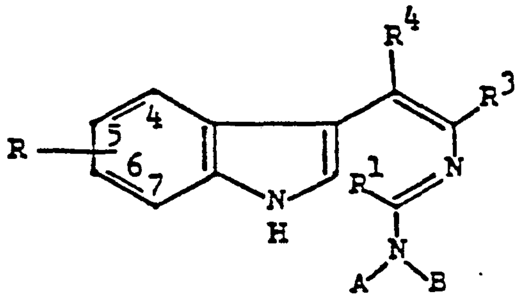

(I)

wherein

R is hydrogen, one or two halogen atoms or alkyl groups of 1 to 4 carbon atoms, alkoxyalkyl groups of 1 to 4 carbon atoms, alkoxy groups of 1 to 4 carbon atoms, methylenedioxy groups, benzyloxy groups, cyano groups, alkoxycarbonyl groups of 1 to 4 carbon atoms in the alkyl part or dialkylamino groups of 1 to 4 carbon atoms in the alkyl part, said groups being in the 4-, 5-, 6- or 7-position,

$R^1$ and $R^4$ are the same or different and are hydrogen, alkyl of 1 to 3 carbon atoms or alkoxyalkyl, with in each case 1 to 3 carbon atoms in the alkoxy and alkyl part.

$R^3$ is phenyl, -COOalkyl, $-PO_3(alkyl)_2$, $-SO_2aryl$, $-SO_2alkyl$,

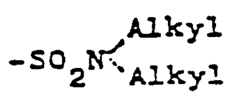 , -CN, 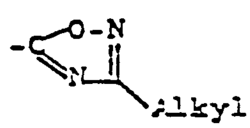 and 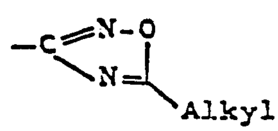

with 1 to 3 carbon atoms in each alkyl group and A and B are each alkyl of 1 to 3 carbon atoms or together with the N form a pyrrolidino, piperidino, morpholino or piperidino group.

2. Ethyl 2-(dimethylaminomethyleneamino)-3-(3-indolyl)-acrylate

3. Ethyl 2-(dimethylaminomethyleneamino)-3-(5-benzyloxy-3-indolyl)acrylate

4. Ethyl 2-(dimethylaminomethyleneamino)-3-(4-cyano-3-indolyl)acrylate

5. Diethyl 2-(dimethylaminomethyleneamino)-2-(3-indolyl)-ethenephosphonate

6. Process for the preparation of indole derivatives of general formula I, characterised in that an indole derivative of general formula II

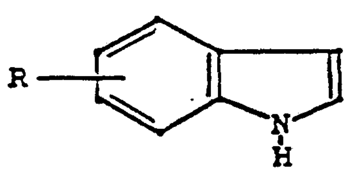

(II)

in which R has the meaning given above, is treated with an azabutadiene of general formula III

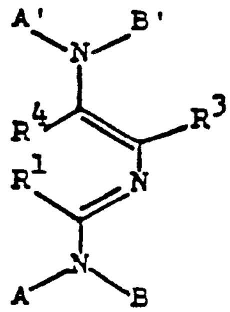

(III)

in which

A' and B' have the same meanings as A and B and $R^1$, $R^3$, $R^4$ and A and B have the meanings given, in the presence of acids at temperatures of 0 to 50° C.

7. Use of the compounds of general formula I according to claim 1 for the preparation of

a) β-carboline derivatives of general formula IV

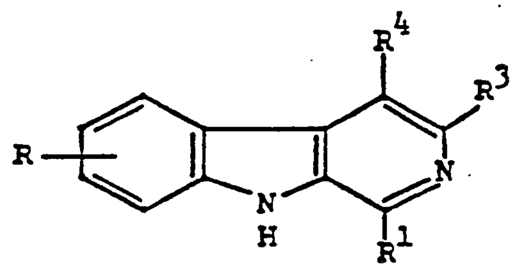

(IV)

in which R, $R^1$, $R^3$ and $R^4$ have the meanings given in formula I, and

b) tryptophan derivatives of general formula V

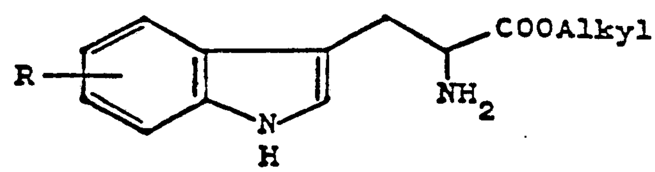

(V)

in which R has the meaning given in formula I.

## Revendications

1. Dérivés de l'indole répondant à la formule générale I:

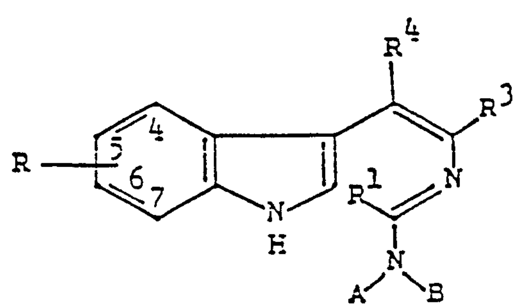

(I)

dans laquelle:

R représente l'hydrogène ou un ou deux substituants pris dans l'ensemble constitué par les atomes d'halogène, les radicaux alkyles contenant de 1 à 4 atomes de carbone, les radicaux alcoxyalkyles en $C_1$-$C_4$, les radicaux alcoxy en $C_1$-$C_4$, le radical méthylène-dioxy, le radical benzyloxy, le radical cyano, les radicaux alcoxycarbonyles à alkyle en $C_1$-$C_4$ et les radicaux dialkylamino dont chacun des alkyles contient de l à 4 atomes de carbone, lesdits substituants pouvant occuper les positions 4, 5, 6 et 7,

$R^1$ et $R^4$ representent chacun, independamment l'un de l'autre, l'hydrogène, un alkyle en $C_1$-$C_3$ ou un alcoxyalkyle dont les parties alcoxy et alkyle contiennent chacune de 1 à 3 atomes de carbone,

$R^3$ represente un radical phényle, -COOAlkyl, $-PO_3(Alkyl)_2$, $-SO_2Aryl$, $-SO_2Alkyl$,

$-SO_2Aryl$, $-SO_2Alkyl$, 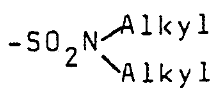, -CN, 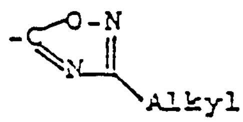 ou

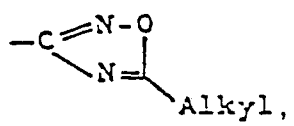

les alkyles contenus dans ces radicaux contenant chacun de 1 à 3 atomes de carbone, et

A et B représentent chacun un alkyle en $C_1$-$C_3$ ou forment ensemble et avec l'atome d'azote un radical pyrrolidino, pipéridino, morpholino ou pipérazino.

2. (Diméthylamino-méthylène-amino)-2 (indolyl-3)-3 acrylate d'éthyle.

3. (Diméthylamino-méthylène-amino)-2 (benzyloxy-5 indolyl-3)-3 acrylate d'éthyle.

4. (Diméthylamino-méthylène-amino)-2 (cyano-4 indolyl-3)-3 acrylate d'éthyle.

5. (Diméthylamino-méthylène-amino)-1 (indolyl-3)-2 éthène-phosphonate de diéthyle.

6. Procédé de préparation de dérivés de l'indole de formule générale I, procédé caractérisé en ce qu'on fait réagir en présence d'acides, à une température de 0 à 50° C, un indole répondant à la formule générale II:

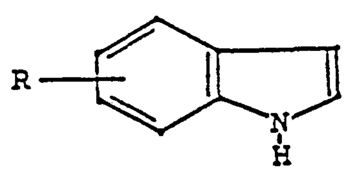

(II)

dans laquelle R a la signification qui a été donnée ci-dessus à propos de la formule I, avec un azabutadiène répondant à la formule générale III:

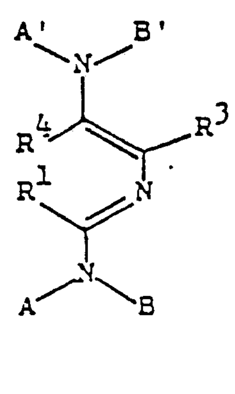

(III)

dans laquelle A' et B' ont chacun la même signification que A et B, et $R^1$, $R^3$, $R^4$, A et B ont les significations qui leur ont été données ci-dessus à propos de la formule I.

7. Application de composés de formule générale I selon la revendication 1 pour la préparation:
a) de dérivés de la β-carboline répondant à la formule générale IV:

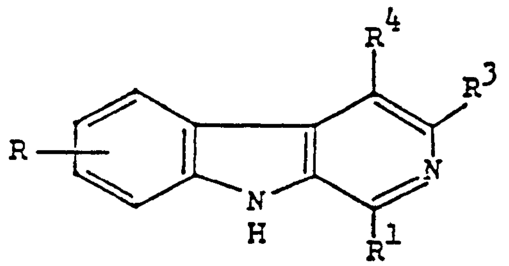

(IV)

dans laquelle R, $R^1$, $R^3$ et $R^4$ ont les significations données à propos de la formule I, et
b) de dérivés du tryptophane répondant à la formule générale V:

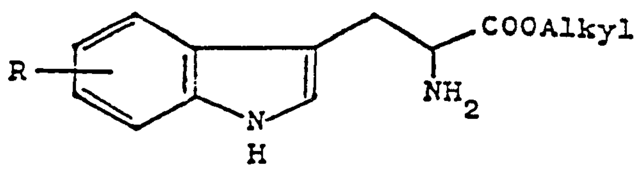

(V)

dans laquelle R a la signification donnée à propos de la formule I.